# EUROPEAN PATENT SPECIFICATION

(11) **EP 2 605 666 B1**
(45) Date of publication and mention of the grant of the patent: **16.02.2022**
(21) Application number: 11817613.0
(22) Date of filing: 18.08.2011
(51) Int. Cl.: A23L 33/185, C07K 1/14, A23J 1/14, A23J 3/16, A23L 2/39, A23L 2/66

(54) **IMPROVED PRODUCTION OF PROTEIN SOLUTIONS FROM SOY**
VERBESSERTE HERSTELLUNG VON PROTEINLÖSUNGEN AUS SOJA
PRODUCTION AMÉLIORÉE DE SOLUTIONS DE PROTÉINE À PARTIR DE SOJA

(30) Priority: 11.04.2011 US 201161457491 P; 26.11.2010 US 344949 P; 18.08.2010 US 344550 P
(43) Date of publication of application: 26.06.2013
(60) Divisional application: 21201754.5
(73) Proprietor: Burcon Nutrascience (MB) Corp., Winnipeg, Manitoba R3T 1P9 (CA)
(72) Inventor: GREEN, Brent E., Warren, Manitoba R0C 3E0 (CA); SCHWEIZER, Martin, Winnipeg, Manitoba R2M 0E3 (CA)
(74) Representative: Beck Greener LLP
(86) International application number: PCT/CA2011/000942
(87) International publication number: WO 2012/021980

(56) References cited:
- WO-A1-2008/041572
- WO-A1-2010/083612
- WO-A1-2011/000096
- US-A1- 2010 098 818
- US-A1- 2010 098 818
- US-A1- 2010 203 204
- US-A1- 2010 330 249
- US-A1- 2010 330 250
- Mark Heyda: "A Practical Guide to Conductivity Measurements", , 29 October 2008 (2008-10-29), Retrieved from the Internet: URL:http://www.mbhes.com/conductivity_meas urement.htm [retrieved on 2017-01-09]

## Description

### REFERENCE TO RELATED APPLICATIONS

This application claims priority under 35 USC 119€ from US Provisional Patent Applications Nos. 61/344,550 filed August 18, 2010, 61/344,949 filed November 26, 2010 and 61/457,491 filed April 11, 2011.

### FIELD OF INVENTION

The present invention relates to the production of protein solutions from soy.

### BACKGROUND TO THE INVENTION

In copending US Patent Application No. 12/603,087 filed October 21, 2009 (US Patent Publication No. 2010-0098818 published April 22, 2010 (S701)), assigned to the assignee hereof, there is described the provision of a novel soy protein product having a protein content of at least about 60 wt% (N x 6.25) on a dry weight basis, preferably a soy protein isolate having a protein content of at least about 90 wt% (N x 6.25) d.b. The soy protein product has a unique combination of properties, namely:
- completely soluble in aqueous media at acid pH values of less than about 4.4
- heat stable in aqueous media at acid pH values of less than about 4.4
- does not require stabilizers or other additives to maintain the protein product in solution
- is low in phytic acid
- requires no enzymes in the production thereof

In addition, the soy protein product has no beany flavour or off odours characteristic of soy protein products.

This soy protein product is prepared by a method which comprises
(a) extracting a soy protein source with an aqueous calcium chloride solution to cause solubilization of soy protein from the protein source and to form an aqueous soy protein solution,
(b) separating the aqueous soy protein solution from residual soy protein source,
(c) optionally diluting the aqueous soy protein solution,
(d) adjusting the pH of the aqueous soy protein solution to a pH of about 1.5 to about 4.4, preferably about 2 to about 4, to produce an acidified clear soy protein solution,
(e) optionally concentrating the aqueous clear soy protein solution while maintaining the ionic strength substantially constant by using a selective membrane technique,
(f) optionally diafiltering the concentrated soy protein solution, and
(g) optionally drying the concentrated soy protein solution.

US 2010/098818 describes the preparation of soy protein solutions having a high clarity of A600 = 0.024 by extracting soy protein source with an aqueous CaCl₂ solution, centrifugation, dilution and acidification. Fine filtration is foreseen. Neither classifying the soy protein source nor a time-dependent dilution step is described.

### SUMMARY OF THE INVENTION

Dalton (Da) refers to unified atomic mass unit (u).

The SI-unit for conductivity is Siemens/meter (S/m). Also mS/cm is used and sometimes abbreviated as mS only in the prior art and also in this application.

M is an abbreviation for mol/l.

It has now been found that the preparation of a soy protein product using this procedure may lead to products that provide aqueous acidic solutions that do not have a preferred level of clarity. It has also been found that measures may be taken to ensure the products do yield aqueous acidic solutions having a preferred level of clarity. The term "a preferred level of clarity" as used herein for an aqueous acidic solution of the protein product is defined as a haze value of less than 10% for a solution prepared by dissolving 3.2 g of protein per 100 ml of water. The haze value is provided by analysis of the aqueous acidic protein solution using a HunterLab Color Quest XE instrument operated in transmission mode.

It has been found that the clarity of the diluted, acidified and optionally heat treated soy protein solution is an indicator of the clarity of an aqueous acidic solution prepared from the soy protein product. Clarity of the diluted, acidifed and optionally heat treated protein solution may be promoted by the use of a fine filtration step in the separation of the aqueous soy protein solution from the residual soy protein source and/or a polishing step, such as fine filtration after the dilution, acidification and optional heat treatment steps. However, fine filtration is an expensive, labour intensive process. Two factors have been found that promote the clarity of the diluted, acidified and optionally heat treated soy protein solution without the use of fine filtration, and result in a preferred level of clarity in aqueous acidic solutions prepared from the soy protein product.

First, it has been found that the speed of the process has an effect on the clarity. It has particularly been found that a preferred level of clarity can be achieved if the dilution and pH adjustment step are carried out within 20 minutes of the separation step in the absence of a subsequent heat treatment step, or within 40 minutes of the separation step if an optional heat treatment step, as discussed below, is employed.

Second, it has been found that, the quantity of fines in the soy protein source may impact the clarity, depending on the extraction and separation conditions. It has particularly been found that under certain extraction and separation conditions, it may be advantageous to reduce the fines content of the soy protein source so that it contains less than about 45% of its weight as particles small enough to pass a 20 mesh sieve.

According to the present invention, there is provided a process for the preparation of a soy protein solution, which comprises:
(a) classifying a soy protein source to provide a classified soy protein source containing less than 45 wt%, preferably less than 35 wt%, of particles small enough to pass a 20 mesh sieve, indicated in US sieve size,
(b) extracting the classified soy protein source material with an aqueous calcium chloride solution having a concentration of from 0.10 to 0.15M at a pH of from 5 to 11 to cause solubilization of soy protein from the classified soy protein source and to form an aqueous soy protein solution,
(c) separating the aqueous soy protein solution from residual soy protein source, and either
(d) within 20 minutes of step (c), (i) diluting the aqueous soy protein solution to a conductivity of less than 90 mS, preferably 4 to 18 mS and (ii) adjusting the pH of the aqueous soy protein solution to a pH of 1.5 to 4.4, preferably 2 to 4 to produce an acidified soy protein solution having an absorbance of visible light at 600 nm (A600) of less than 0.055, preferably less than 0.040, or
(e) within 40 minutes of step (c), (i) diluting the aqueous soy protein solution to a conductivity of less than 90 mS, preferably 4 to 18 mS, (ii) adjusting the pH of the aqueous soy protein solution to a pH of 1.5 to 4.4, preferably 2 to 4 and (iii) heat treating

the aqueous protein solution at a temperature of 70° to 160°C for 10 seconds to 60 minutes, preferably 80° to 120°C for 10 seconds to 5 minutes, more preferably 85° to 95°C for 30 seconds to 5 minutes to produce an acidified soy protein solution having an absorbance of visible light at 600 nm (A600) of less than 0.055, preferably less than 0.040;
wherein the process is carried out without the use of fine filtration.

It has further been found that benefits can be obtained if the extraction and separation steps as well as the subsequent steps of dilution and/or membrane processing, are effected at an elevated temperature, generally 35° to 65°C, preferably 50° to 60°C, in comparison to effecting these steps at ambient temperatures of about 20° to about 25°C. Effecting the extraction and separation steps at elevated temperature provides an aqueous soy protein solution with better clarity than if the same steps were effected at ambient temperature. It is advantageous to maximize the clarity of the aqueous soy protein solution resulting from the separation step in order to provide a final product with a minimal haze when resolubilized. Extraction and separation at elevated temperature is also beneficial in that it permits soy meal or other protein sources with higher fines content to be processed and still yield soy protein product which provides aqueous acidic solutions having a preferred level of clarity. Effecting the membrane processing at an elevated temperature results in greater removal of trypsin inhibitors and greater flux rates compared to effecting the processing at ambient temperature. Effecting all of the indicated processing steps at the higher end of the temperature range (e.g. 50° to 65°C) may result in a lower microbial load than if the steps were effected at ambient temperature.

The soy protein product produced according to the process (product not falling under the scope of the claimed invention) herein lacks the characteristic beany flavour of soy protein products and is suitable, not only for protein fortification of acid media, but may be used (uses not falling under the scope of the claimed invention) in a wide variety of conventional applications of protein products, including but not limited to protein fortification of processed foods and beverages, emulsification of oils, as a body former in baked goods and foaming agent in products which entrap gases. In addition, the soy protein product may be formed into protein fibers, useful in meat analogs and may be used as an egg white substitute or extender in food products where egg white is used as a binder. The soy protein product may be used in nutritional supplements. The soy protein product may also be used in dairy analogue products or products that are dairy/soy blends. Other uses of the soy protein product are in pet foods, animal feed and in industrial and cosmetic applications and in personal care products.

### GENERAL DESCRIPTION OF INVENTION

The soy protein source utilized in the process may be soybeans or any soy product or by-product derived from the processing of soybeans, including but not limited to soy meal, soy flakes, soy grits and soy flour. The soy protein source may be used in the full fat form, partially defatted form or fully defatted form. Where the soy protein source contains an appreciable amount of fat, an oil-removal step generally is required during the process. The soy protein recovered from the soy protein source may be a protein modified by genetic manipulation but possessing characteristic hydrophobic and polar properties of the natural protein.

Prior to the protein solubilization step, the soy protein source is classified to reduce the fines content. The soy protein source is classified so that it contain less than 45 wt%, preferably less than 35 wt% of fines that pass through a 20 mesh sieve, indicated in US sieve size.

The classification may be conveniently effected by sieving the soy protein source.

Protein solubilization from the classified soy protein source material is effected using calcium chloride solutions as set forth in claim 1. In addition, other alkaline earth metal compounds may be used, such as magnesium salts. Further, extraction of the soy protein from the soy protein source may be effected using said calcium salt solution in combination with another salt solution, such as sodium chloride.

Precipitate formed upon addition of the said calcium salt is removed prior to subsequent processing.

As the concentration of the calcium salt solution increases, the degree of solubilization of protein from the soy protein source initially increases until a maximum value is achieved. Any subsequent increase in salt concentration does not increase the total protein solubilized. The concentration of calcium salt solution which causes maximum protein solubilization varies depending on the salt concerned. A concentration value of 0.10 to 0.15 M is used.

In a batch process, the salt solubilization of the protein according to claim 1 is effected at a temperature of from 1°C to 100°C, preferably 15°C to 65°C, more preferably 50°C to 60°C, preferably accompanied by agitation to decrease the solubilization time, which is usually 1 to 60 minutes. It is preferred to effect the solubilization to extract substantially as much protein from the soy protein source as is practicable, so as to provide an overall high product yield.

In a continuous process, the extraction of the soy protein from the soy protein source is carried out in any manner consistent with effecting a continuous extraction of soy protein from the soy protein source. In one embodiment, the soy protein source is continuously mixed with the calcium salt solution according to claim 1 and the mixture is conveyed through a pipe or conduit having a length and at a flow rate for a residence time sufficient to effect the desired extraction in accordance with the parameters described herein. In such a continuous procedure, the salt solubilization step according to claim 1 is effected rapidly, in a time of up to about 10 minutes, preferably to effect solubilization to extract substantially as much protein from the soy protein source as is practicable. The solubilization in the continuous procedure is effected at temperatures between 1°C and 100°C, preferably 15°C to 65°C, more preferably between 50°C and 60°C.

The extraction is conducted at a pH of 5 to 11, preferably 5 to 7. The pH of the extraction system (soy protein source and calcium salt solution) may be adjusted to any desired value within the range of 5 to 11 for use in the extraction step by the use of any convenient food grade acid, usually hydrochloric acid or phosphoric acid, or food grade alkali, usually sodium hydroxide, as required.

The concentration of soy protein source in the calcium salt solution during the solubilization step may vary widely. Typical concentration values are about 5 to about 15% w/v.

The protein extraction step with the aqueous salt solution has the additional effect of solubilizing fats which may be present in the soy protein source, which then results in the fats being present in the aqueous phase.

The protein solution resulting from the extraction step generally has a protein concentration of 5 to 50 g/L, preferably 10 to 50 g/L.

The aqueous calcium salt solution may contain an antioxidant. The antioxidant may be any convenient antioxidant, such as sodium sulfite or ascorbic acid. The quantity of antioxidant employed may vary from about 0.01 to about 1 wt% of the solution, preferably about 0.05 wt%. The antioxidant serves to inhibit oxidation of any phenolics in the protein solution.

The aqueous phase resulting from the extraction step then may be separated from the residual soy protein source, in any convenient manner, such as by employing a decanter centrifuge or any suitable sieve, followed by disc centrifugation and/or filtration, to remove residual soy protein source material. The separation step is generally conducted at the same temperature as the protein solubilization step, but may be conducted at any temperature within the range of 1° to 100°C, preferably 15° to 65°C, more preferably 50° to 60°C. The separated residual soy protein source (not falling under the scope of the claimed invention) may be dried for disposal. Alternatively, the separated residual soy protein source may be processed to recover some residual protein. The separated residual soy protein source may be re-extracted with fresh calcium salt solution and the protein solution yielded upon clarification combined with the initial protein solution for further processing as described below. Alternatively, the separated residual soy protein source may be processed by a conventional isoelectric precipitation procedure or any other convenient procedure to recover residual protein.

As noted above, it may be preferred to effect the extraction step, whether in a batch or continuous operation, and subsequent step of separation of aqueous soy protein solution from residual soy protein source, at an elevated temperature, generally 35° to 65°C, preferably 50° to 60°C, to result in a clarified soy protein solution having an improved clarity, compared to effecting the steps at ambient temperature.

Where the soy protein source contains significant quantities of fat, as described in US Patents Nos. 5,844,086 and 6,005,076, assigned to the assignee hereof, then the defatting steps described therein may be effected on the separated aqueous protein solution. Alternatively, defatting of the separated aqueous protein solution may be achieved by any other convenient procedure.

The aqueous soy protein solution may be treated with an adsorbent, such as powdered activated carbon or granulated activated carbon, to remove colour and/or odour compounds. Such adsorbent treatment may be carried out under any convenient conditions, generally at the ambient temperature of the separated aqueous protein solution. For powdered activated carbon, an amount of about 0.025% to about 5% w/v, preferably about 0.05% to about 2% w/v, is employed. The adsorbing agent may be removed from the soy solution by any convenient means, such as by filtration.

The resulting aqueous soy protein solution is diluted generally with about 0.5 to about 10 volumes, preferably about 0.5 to about 2 volumes of aqueous diluent, in order to decrease the conductivity of the aqueous soy protein solution to a value of below 90 mS, preferably 4 to 18 mS. Such dilution is usually effected using water although dilute salt solution such as sodium chloride or calcium chloride, having a conductivity of up to about 3 mS, may be used.

The diluent with which the soy protein solution is mixed generally has the same temperature as the soy protein solution, but the diluent may have a temperature of 1° to 100°C, preferably 15° to 65°C, more preferably 50° to 60°C.

The diluted soy protein solution then is adjusted in pH to a value of 1.5 to 4.4, preferably 2 to 4, by the addition of any suitable food grade acid, such as hydrochloric acid or phosphoric acid, to result in a clear acidified aqueous soy protein solution.

The clear acidified aqueous soy protein solution has a conductivity of generally below about 95 mS, preferably about 4 to about 23 mS.

The absorbance at 600 nm of the diluted and acidified solution is an important indicator of the clarity of the acidic solution of reconstituted soy protein product. An absorbance at 600 nm (A600) of the diluted and acidified solution of less than 0.055, preferably less than 0.040, is critical to achieving a final product that provides an aqueous acidic solution that meets the definition of a preferred level of clarity set forth above. When the optional heat treatment step described below is employed, the clarity of the protein solution has been found to improve. Therefore, an A600 value for the diluted and acidified solution of greater than 0.055 may be acceptable if the subsequent heat treatment step is employed and the heat treatment improves the clarity sufficiently that the A600 of the diluted, acidified and heat-treated solution is less than 0.055, preferably less than 0.040.

It has been found that process speed is important to ensure that solutions having a preferred level of clarity can be provided from the soy protein product of the process. The longer the protein extract solution remains at natural pH after removal of the residual soy protein source, the hazier the solution becomes. This haze formation occurs no matter the clarity of the aqueous soy protein solution resulting from the separation step. A completely haze-free solution becomes hazy if not diluted and acidified in short order. After the haze forms, it is very difficult to remove, even through the use of fine filtration.

It is beneficial, therefore, to perform the dilution and acidification as quickly as possible, if not immediately, after the extract solution has been clarified. Testing has shown that the maximum amount of time between clarification and dilution and acidification should be 20 minutes or 40 minutes if the optional heat treatment is to be employed, with a shorter time more desirable and immediate dilution and acidification being optimal.

The clear acidified aqueous soy protein solution may be subjected to a heat treatment to inactivate heat labile anti-nutritional factors, such as trypsin inhibitors, present in such solution as a result of extraction from the soy protein source material during the extraction step. Such a heating step also provides the additional benefits of reducing the microbial load and improving the clarity of the solution. An absorbance at 600 nm (A600) of the diluted, acidified and heat treated solution of less than 0.055, preferably less than 0.040, is critical to achieving a final product that provides an aqueous acidic solution that meets the definition of a preferred level of clarity. The protein solution is heated to a temperature of 70° to 160°C for 10 seconds to 60 minutes, preferably 80° to 120°C for 10 seconds to 5 minutes, more preferably 85° to 95°C for 30 seconds to 5 minutes. The heat treated acidified soy protein solution then may be cooled for further processing as described below, to a temperature of 2° to 65°C, preferably 50°C to 60°C.

The resulting clear acidified aqueous soy protein solution may be directly dried to produce a soy protein product (product not falling under the scope of the claimed invention). In order to provide a soy protein product having a decreased impurities content and a reduced salt content, such as a soy protein isolate, the clear acidified aqueous soy protein solution may be processed prior to drying.

The clear acidified aqueous soy protein solution may be concentrated to increase the protein concentration thereof while maintaining the ionic strength thereof substantially constant. Such concentration generally is effected to provide a concentrated soy protein solution having a protein concentration of 50 to 300 g/L, preferably 100 to 200 g/L.

The concentration step may be effected in any convenient manner consistent with batch or continuous operation, such as by employing any convenient selective membrane technique, such as ultrafiltration or diafiltration, using membranes, such as hollow-fibre membranes or spiral-wound membranes, with a suitable molecular weight cut-off, such as 3,000 to 1,000,000 Daltons, preferably 5,000 to 100,000 Daltons, having regard to differing membrane materials and configurations, and, for continuous operation, dimensioned to permit the desired degree of concentration as the aqueous protein solution passes through the membranes.

As is well known, ultrafiltration and similar selective membrane techniques permit low molecular weight species to pass therethrough while preventing higher molecular weight species from so doing. The low molecular weight species include not only the ionic species of the food grade salt but also low molecular weight materials extracted from the source material, such as carbohydrates, pigments, low molecular weight proteins and anti-nutritional factors, such as trypsin inhibitors, which are themselves low molecular weight proteins. The molecular weight cut-off of the membrane is usually chosen to ensure retention of a significant proportion of the protein in the solution, while permitting contaminants to pass through having regard to the different membrane materials and configurations.

The concentrated soy protein solution then may be subjected to a diafiltration step using water or a dilute saline solution. The diafiltration solution may be at its natural pH or at a pH equal to that of the protein solution being diafiltered or at any pH value in between. Such diafiltration may be effected using from 1 to 40 volumes of diafiltration solution, preferably 2 to 25 volumes of diafiltration solution. In the diafiltration operation, further quantities of contaminants are removed from the clear aqueous soy protein solution by passage through the membrane with the permeate. This purifies the clear aqueous protein solution and may also reduce its viscosity. The diafiltration operation may be effected until no significant further quantities of contaminants or visible colour are present in the permeate or until the retentate has been sufficiently purified so as, when dried, to provide a soy protein isolate with a protein content of at least 90 wt% (N x 6.25) d.b. Such diafiltration may be effected using the same membrane as for the concentration step. However, if desired, the diafiltration step may be effected using a separate membrane with a different molecular weight cut-off, such as a membrane having a molecular weight cut-off in the range of 3,000 to 1,000,000 Daltons, preferably 5,000 to 100,000 Daltons, having regard to different membrane materials and configuration.

Alternatively, the diafiltration step may be applied to the clear acidified aqueous protein solution prior to concentration or to the partially concentrated clear acidified aqueous protein solution. Diafiltration may also be applied at multiple points during the concentration process. When diafiltration is applied prior to concentration or to the partially concentrated solution, the resulting diafiltered solution may then be additionally concentrated. The viscosity reduction achieved by diafiltering multiple times as the protein solution is concentrated may allow a higher final, fully concentrated protein concentration to be achieved. This reduces the volume of material to be dried.

The concentration step and the diafiltration step may be effected herein in such a manner that the soy protein product subsequently recovered contains less than 90 wt% protein (N x 6.25) d.b., such as at least 60 wt% protein (N x 6.25) d.b. By partially concentrating and/or partially diafiltering the clear aqueous soy protein solution, it is possible to only partially remove contaminants. This protein solution may then be dried to provide a soy protein product with lower levels of purity. The soy protein product having a protein content of at least 60 wt% is still able to produce clear protein solutions under acidic conditions.

An antioxidant may be present in the diafiltration medium during at least part of the diafiltration step. The antioxidant may be any convenient antioxidant, such as sodium sulfite or ascorbic acid. The quantity of antioxidant employed in the diafiltration medium depends on the materials employed and may vary from about 0.01 to about 1 wt%, preferably about 0.05 wt%. The antioxidant serves to inhibit the oxidation of any phenolics present in the concentrated soy protein solution.

The concentration step and the optional diafiltration step may be effected at any convenient temperature, generally 2° to 65°C, preferably 50° to 60°C, and for the period of time to effect the desired degree of concentration and diafiltration. The temperature and other conditions used to some degree depend upon the membrane equipment used to effect the membrane processing, the desired protein concentration of the solution and the efficiency of the removal of contaminants to the permeate.

There are two main trypsin inhibitors in soy, namely the Kunitz inhibitor, which is a heat-labile molecule with a molecular weight of approximately 21,000 Daltons, and the Bowman-Birk inhibitor, a more heat-stable molecule with a molecular weight of about 8,000 Daltons. The level of trypsin inhibitor activity in the final soy protein product can be controlled by manipulation of various process variables.

As noted above, heat treatment of the clear acidified aqueous soy protein solution may be used to inactivate heat-labile trypsin inhibitors. The partially concentrated or fully concentrated acidified soy protein solution may also be heat treated to inactivate heat labile trypsin inhibitors. When the heat treatment is applied to the partially concentrated acidified soy protein solution, the resulting heat treated solution then may be additionally concentrated.

In addition, the concentration and/or diafiltration steps may be operated in a manner favorable for removal of trypsin inhibitors in the permeate along with the other contaminants. Removal of the trypsin inhibitors is promoted by using a membrane of larger pore size, such as 30,000 to 1,000,000 Daltons, operating the membrane at elevated temperatures such as 30° to 65°C, preferably 50° to 60°C and employing greater volumes of diafiltration medium, such as 10 to 40 volumes.

Acidifying and membrane processing the diluted protein solution at a lower pH of 1.5 to 3 may reduce the trypsin inhibitor activity relative to processing the solution at a higher pH of 3 to 4.4. When the protein solution is concentrated and diafiltered at the low end of the pH range, it may be desired to raise the pH of the retentate prior to drying. The pH of the concentrated and diafiltered protein solution may be raised to the desired value, for example pH 3, by the addition of any convenient food grade alkali such as sodium hydroxide.

Further, a reduction in trypsin inhibitor activity may be achieved by exposing soy materials to reducing agents that disrupt or rearrange the disulfide bonds of the inhibitors. Suitable reducing agents include sodium sulfite, cysteine and N-acetylcysteine.

The addition of such reducing agents may be effected at various stages of the overall process. The reducing agent may be added with the soy protein source material in the extraction step, may be added to the clarified aqueous soy protein solution following removal of residual soy protein source material, may be added to the concentrated protein solution before or after diafiltration or may be dry blended with the dried soy protein product. The addition of the reducing agent may be combined with a heat treatment step and the membrane processing steps, as described above.

If it is desired to retain active trypsin inhibitors in the concentrated protein solution, this can be achieved by eliminating or reducing the intensity of the heat treatment step, not utilizing reducing agents, operating the concentration and diafiltration steps at the higher end of the pH range, such as pH 3 to 4.4, utilizing a concentration and diafiltration membrane with a smaller pore size, operating the membrane at lower temperatures and employing fewer volumes of diafiltration medium.

The concentrated and optionally diafiltered protein solution may be subject to a further defatting operation, if required, as described in US Patents Nos. 5,844,086 and 6,005,076. Alternatively, defatting of the concentrated and optionally diafiltered protein solution may be achieved by any other convenient procedure.

The concentrated and optionally diafiltered clear aqueous protein solution may be treated with an adsorbent, such as powdered activated carbon or granulated activated carbon, to remove colour and/or odour compounds. Such adsorbent treatment may be carried out under any convenient conditions, generally at the ambient temperature of the concentrated protein solution. For powdered activated carbon, an amount of about 0.025% to about 5% w/v, preferably about 0.05% to about 2% w/v, is employed. The adsorbent may be removed from the soy protein solution by any convenient means, such as by filtration.

The concentrated and optionally diafiltered clear aqueous soy protein solution may be dried by any convenient technique, such as spray drying or freeze drying. A pasteurization step may be effected on the soy protein solution prior to drying. Such pasteurization may be effected under any desired pasteurization conditions. Generally, the concentrated and optionally diafiltered soy protein solution is heated to a temperature of about 55° to about 70°C, preferably about 60° to about 65°C, for about 30 seconds to about 60 minutes, preferably about 10 minutes to about 15 minutes. The pasteurized concentrated soy protein solution then may be cooled for drying, preferably to a temperature of about 25° to about 40°C.

The dry soy protein product (product not falling under the scope of the claimed invention) has a protein content in excess of 60 wt% (N x 6.25) d.b. Preferably, the dry soy protein product is an isolate with a high protein content, in excess of 90 wt% protein, preferably at least 100 wt%, (N x 6.25) d.b..

The soy protein product produced herein (product not falling under the scope of the claimed invention) is soluble in an acidic aqueous environment, making the product ideal for incorporation into beverages, both carbonated and uncarbonated, to provide protein fortification thereto. Such beverages have a wide range of acidic pH values, ranging from about 2.5 to about 5. The soy protein product provided herein may be added to such beverages in any convenient quantity to provide protein fortification to such beverages, for example, at least about 5 g of the soy protein per serving. The added soy protein product dissolves in the beverage and does not impair the clarity of the beverage, even after thermal processing. The soy protein product may be blended with dried beverage prior to reconstitution of the beverage by dissolution in water. In some cases, modification to the normal formulation of the beverages to tolerate the inclusion of the soy protein product may be necessary where components present in the beverage may adversely affect the ability of the soy protein product to remain dissolved in the beverage.

### EXAMPLES

Example 1 (Example not according to the present invention)

This Example illustrates the effect of fines content in a soy protein source on the clarity of both the diluted, acidified and heat treated soy protein solution and an aqueous acidic solution of the final soy protein product.

30 kg of defatted soy white flake containing 'a' % of the weight of the flake as fines passing a 20 mesh sieve was added to 'b' L of 0.15 M CaCl₂ solution at ambient temperature and agitated for 30 minutes to provide an aqueous protein solution. The residual soy white flake was removed and the resulting protein solution was clarified by centrifugation to provide 'c' L of protein solution having a protein content of 'd' % by weight and an absorbance at 600 nm of 'e'.

The protein solution was then added to 'f volume(s) of reverse osmosis purified water and the pH of the sample lowered to 'g' with diluted 'h' (one volume concentrated 'h' plus one volume water). The diluted and acidified solution was then heat treated at 90°C for 30 seconds. The A600 of the protein solution after heat treatment was 'i'.

The heat treated acidified protein solution was reduced in volume from 'j' L to 'k' L by concentration on a polyethersulfone membrane, having a molecular weight cutoff of 100,000 Daltons, operated at a temperature of approximately 'l' °C. The acidified protein solution, with a protein content of 'm' wt %, was diafiltered with 'n' L of reverse osmosis (RO) purified water, with the diafiltration operation conducted at approximately 'o' °C. The diafiltered solution was then further concentrated to a volume of 'p' L and diafiltered with an additional 'q' L of RO water, with the diafiltration operation conducted at approximately 'r' °C. After this second diafiltration, the protein solution was concentrated from a protein content of 's' to a protein content of 't' % by weight then diluted to a protein content of 'u' % by weight with water to facilitate spray drying. The protein solution before spray drying was recovered in a yield of 'v' wt% of the initial centrifuged protein solution. The acidified, diafiltered, concentrated protein solution was then dried to yield a product found to have a protein content of 'w'% (N x 6.25) d.b. The product was given designation 'x' S701H. A solution of the S701H was prepared by dissolving sufficient protein powder to supply 0.48 g of protein in 15 ml of RO water and had a pH of 'y'. The haze value for this solution was determined using a HunterLab Color Quest XE instrument operated in transmission mode and found to be 'z'.

The parameters 'a' to 'z' for thirteen runs are set forth in the following Table 1:

**Table 1 - Parameters for the runs to produce S701H**

| x | S013-J29-09A | S013-K05-09A | S013-K12-09A | S016-K23-09A | S015-A14-10A | S017-B18-10A | S014-B24-10A | S017-C10-10A | S017-D13-10A | S019-D15-10A | S019-D20-10A | S019-D20-10A | S019-D21-10A |
|---|---|---|---|---|---|---|---|---|---|---|---|---|---|
| a | 44.3 | 44.3 | 44.3 | 33 | 45.8 | 48 | 44.6 | 48 | 52.7 | 30.7 | 30.7 | 30.7 | 30.7 |
| b | 300 | 300 | 300 | 300 | 300 | 400 | 300 | 300 | 300 | 300 | 300 | 300 | 300 |
| c | 250 | 269 | 230.5 | 273 | 251 | 306.3 | 232 | 230 | 219.6 | 209.3 | 233 | 228 | 221 |
| d | 2.19 | 2.61 | 2.14 | 2.27 | 2.52 | 2.20 | 2.74 | 2.79 | 2.76 | 2.76 | 2.83 | 2.69 | 2.79 |
| e | 0.216 | 0.454 | 0.166 | 0.202 | 0.435 | 0.298 | 0.264 | 0.343 | 0.248 | 0.251 | 0.377 | 0.248 | 0.271 |
| f | 1 | 1 | 1 | 1 | 0.75 | 1 | 1 | 1 | 1 | 1 | 1 | 1 | 1 |
| g | 2.66 | 2.99 | 2.95 | 3.08 | 2.57 | 3.19 | 3.03 | 2.82 | 3.18 | 3.27 | 3.14 | 3.05 | 3.29 |
| h | H₃PO₄ | HCl | HCl | HCl | HCl | HCl | HCl | HCl | HCl | HCl | HCl | HCl | HCl |
| i | 0.034 | 0.041 | 0.039 | 0.026 | 0.056 | 0.056 | 0.022 | 0.060 | 0.061 | 0.027 | 0.013 | 0.016 | 0.023 |
| j | 510 | 535 | 475 | 518 | 390 | 670 | 465 | 503 | 475 | 408 | 485 | 500 | 480 |
| k | 95 | 100 | 82 | 107 | 88 | 120 | 100 | 115 | 117 | 89 | 96 | 108 | 107 |
| l | 30 | 30 | 29 | 29 | 30 | 31 | 30 | 30 | 30 | 30 | 50 | 29 | 50 |
| m | 4.53 | 5.30 | 5.15 | 4.53 | 6.25 | 4.28 | 4.70 | 4.95 | 4.53 | 4.99 | 5.81 | 4.77 | 4.73 |
| n | 120 | 125 | 123 | 160.5 | 132 | 180 | 150 | 173 | 176 | 134 | 144 | 162 | 160 |
| o | 29 | 30 | 30 | 31 | 30 | 30 | 30 | 30 | 30 | 30 | 51 | 29 | 50 |
| p | 48 | 50 | 41 | 54 | 44 | 60 | 42 | 51 | 49 | 41 | 48 | 47 | 48 |
| q | 365 | 375 | 307.5 | 405 | 330 | 450 | 315 | 383 | 368 | 308 | 360 | 353 | 360 |
| r | 29 | 30 | 30 | 30 | 31 | 30 | 30 | 30 | 30 | 30 | 49 | 30 | 51 |
| s | 8.33 | 9.02 | 9.91 | 7.92 | 10.87 | 9.38 | 9.69 | 10.51 | 9.87 | 9.66 | 10.85 | 9.89 | 9.34 |
| t | N/A | N/A | N/A | N/A | N/A | N/A | N/A | 12.42 | 12.29 | 11.78 | 13.49 | 11.78 | 11.86 |
| u | N/A | N/A | N/A | N/A | N/A | 8.62 | 8.17 | 5.78 | 5.71 | 5.94 | 6.22 | 5.02 | 5.55 |
| v | 77.6 | 65.1 | 81.9 | 66.8 | 73.9 | 79.1 | 64.8 | 83.0 | 77.2 | 74.0 | 79.2 | 66.4 | 77.3 |
| w | 100.67 | 101.68 | 100.24 | 100.95 | 102.61 | 102.25 | 101.56 | 101.07 | 100.62 | 100.53 | 102.43 | 102.10 | 102.45 |
| y | 3.29 | 3.38 | 3.42 | 3.47 | 3.05 | 3.19 | 3.51 | 3.32 | 3.42 | 3.22 | 3.25 | 3.42 | 3.45 |
| z | 3.1 | 9.7 | 5.5 | 5.9 | 16.8 | 23.6 | 9.2 | 16.7 | 12.1 | 5.4 | 1.8 | 3.8 | 2.8 |

| | | | | | | | | | | | | | |
|---|---|---|---|---|---|---|---|---|---|---|---|---|---|
| N/A means not applicable | | | | | | | | | | | | | |

As can be seen from the data contained in Table 1, samples of soy white flake containing less than about 45 wt%, preferably less than about 35 wt%, of fines passing a 20 mesh sieve provided diluted, acidified and heat treated protein solutions having an A600 of less than about 0.055, preferably less than about 0.040, and aqueous acidic solutions of soy protein isolate having a preferred haze level of less than 10%.

### Example 2 (Example not according to the present invention)

This Example shows the effect of process speed on the clarity of the diluted, acidified and heat treated protein solution.

60 kg of defatted soy white flake was added to 600 L of 0.15 M CaCl₂ solution at ambient temperature and agitated for 30 minutes to provide an aqueous protein solution. The residual soy white flake was removed and the resulting protein solution was clarified by centrifugation to provide 473.5 L of protein solution having a protein content of 2.75% by weight and an A600 value of 'a'.

The protein solution was then added to 1 volume of reverse osmosis purified water and the pH of the sample lowered to 3 with diluted HCl (one volume concentrated HCl plus one volume water). The A600 of the protein solution after dilution and pH adjustment was 'b'. The diluted and acidified solution was then heat treated at 90°C for 30 seconds. The A600 of the protein solution after heat treatment was 'c'.

The procedure was repeated with samples of centrate that were taken every 10 minutes after separation from the residual soy white flakes, up to 70 minutes. The results obtained are set forth in the following Table 2:

**Table 2: Effect of time between clarification and further processing on solution clarity**

| Time between clarification and dilution/pH adiustment/heat treatment | 'a' | 'b' | 'c' |
|---|---|---|---|
| Immediate dilution and pH adjustment | 0.225 | 0.043 | 0.037 |
| 10 Minutes | 0.252 | 0.050 | 0.047 |
| 20 Minutes | 0.280 | 0.053 | 0.046 |
| 30 Minutes | 0.310 | 0.058 | 0.050 |
| 40 Minutes | 0.325 | 0.068 | 0.053 |
| 50 Minutes | 0.338 | 0.069 | 0.056 |
| 60 Minutes | 0.362 | 0.087 | 0.059 |
| 70 Minutes | 0.378 | 0.094 | 0.067 |

From the data presented in Table 2, the maximum recommended time between clarification and the subsequent steps of dilution and acidification is about 20 minutes if the solution is to be further processed without heat treatment or 40 minutes if a subsequent heat treatment step is employed. Over these times the A600 of the protein solution may be higher than the value (0.055) determined to indicate an aqueous acidic solution of the final soy protein product having a haze value below 10%. As can be seen from Table 2, the sooner the clarified extract is further processed, the greater the clarity of the diluted, acidified and optionally heat treated protein solution.

### Example 3 (Example not according to the present invention)

This Example illustrates the effect of temperature on clarity.

A comparison was made with respect to post desludger centrate clarity, as expressed as the absorbance of visible light at 600 nm (A600) for each of eight process runs. In each run, 300 L of 0.15M CaCl₂ was used to extract 30 kg of soybean meal for 30 minutes. Each extraction was passed through a decanter to remove the residual soy protein source and then through a disc stack desludger centrifuge to further clarify the solution. Four of the batches were processed at approximately 50°C while the other four batches were processed at an ambient temperature of approximately 20° to 25°C.

The A600 readings for a sample of the post desludger centrate were recorded for each batch and the results obtained are set forth in the following Table 3:

**Table 3**

| 50°C | | Ambient Temperature | |
|---|---|---|---|
| Batch | Centrate A600 | Batch | Centrate A600 |
| BW-S020-H09-10A | 0.084 | BW-S020-G20-10A | 0.385 |
| BW-S020-H16-10A | 0.070 | BW-S020-H03-10A | 0.375 |
| BW-S020-H31-10A | 0.133 | BW-S020-H23-10A | 0.461 |
| BW-S020-I16-10A | 0.120 | BW-S020-H26-10A | 0.375 |

As may be seen from the results of Table 3, significantly lower haze, as determined by A600 values, was exhibited in the batches that were extracted and clarified at approximately 50°C.

### SUMMARY OF THE DISCLOSURE

In summary of this disclosure, the present invention is concerned with a process comprising at least the processing steps as set forth in claim 1 which ensure the production of a soy protein product (not falling under the scope of the claimed invention) which can be taken into acidic solution and provide a preferred level of clarity.

## Claims

1. A process for the preparation of a soy protein solution, which comprises:
(a) classifying a soy protein source to provide a soy protein source containing less than 45 wt%, preferably less than 35 wt%, of particles small enough to pass a 20 mesh sieve, indicated in US sieve size,
(b) extracting the classified soy protein source with an aqueous calcium chloride solution having a concentration of 0.10 to 0.15 mol/l (M) at a pH of 5 to 11 to cause solubilization of soy protein from the classified soy protein source and to form an aqueous soy protein solution,
(c) separating the aqueous soy protein solution from residual soy protein source, and either
(d) within 20 minutes of step (c), (i) diluting the aqueous soy protein solution to a conductivity of less than 90 mS/cm (also commonly abbreviated mS), preferably 4 to 18 mS/cm (also commonly abbreviated mS), and (ii) adjusting the pH of the aqueous soy protein solution to a pH of 1.5 to 4.4, preferably 2 to 4, to produce an acidified soy protein solution having an absorbance of visible light at 600 nm (A600) of less than 0.055, preferably less than 0.040, or
(e) within 40 minutes of step (c), (i) diluting the aqueous soy protein solution to a conductivity of less than 90 mS/cm (also commonly abbreviated mS), preferably 4 to 18 mS/cm (also commonly abbreviated mS), (ii) adjusting the pH of the
aqueous soy protein solution to a pH of 1.5 to 4.4, preferably 2 to 4, and (iii) heat treating the aqueous protein solution at a temperature of 70° to 160°C for 10 seconds to 60 minutes to produce an acidified soy protein solution having an absorbance of visible light at 600 nm (A600) of less than 0.055;
wherein the process is carried out without the use of fine filtration.

2. The process claimed in claim 1, wherein said extraction of the soy protein source is effected at a temperature of 1° to 100°C, preferably 15° to 65°C, more preferably 50° to 60°C, and/or said extraction of the soy protein source is effected at a pH of 5 to 7, and/or said aqueous soy protein solution produced in the extraction step has a protein concentration of 5 to 50 g/L.

3. The process claimed in claim 1 or claim 2, wherein said separation step is effected at a temperature of 1° to 100°C, preferably 15° to 65°C, more preferably 50° to 60°C, or said extraction step and said separation step are effected at a temperature of 50° to 60°C.

4. The process claimed in any one of claims 1 to 3, wherein said aqueous soy protein solution is diluted with water at a temperature of 1° to 100°C, preferably 15° to 65°C, more preferably 50° to 60°C, or said extraction step, said separation step, said dilution step and said acidification step are effected at a temperature of 50° to 60°C.

5. The process claimed in any one of claims 1 to 4, wherein said heat treatment of the aqueous protein solution is effected at a temperature of 80° to 120°C for 10 seconds to 5 minutes, preferably at a temperature of 85° to 95°C for 30 seconds to 5 minutes.

6. The process claimed in any one of claims 1 to 5, wherein the acidified aqueous soy protein solution produced in step (e) is cooled for further processing to a temperature of 2° to 65°C, preferably 50°C to 60°C.

7. The process claimed in any one of claims 1 to 6, wherein the acidified aqueous soy protein solution is dried to provide a soy protein product.

8. The process claimed in any one of claims 1 to 7, wherein the acidified aqueous soy protein solution is concentrated while maintaining the ionic strength thereof substantially constant to provide a concentrated soy protein solution having a protein concentration of 50 to 300 g/L, preferably 100 to 200 g/L, preferably using membranes having a molecular weight cut-off of 3,000 to 1,000,000 unified atomic mass units (u = Dalton) preferably 5,000 to 100,000 Daltons, and the concentrated soy protein solution is optionally subjected to diafiltration using water or a dilute saline solution, preferably using 1 to 40 volumes, preferably 2 to 25 volumes, of diafiltration solution, preferably effected until no significant further quantities of contaminants or visible colour are present in the permeate or until the retentate has been sufficiently purified so as, when dried, to provide a soy protein isolate with a protein content of at least 90 wt% (N x 6.25), using membranes having a molecular weight cut-off of 3,000 to 1,000,000 Daltons, preferably 5,000 to 100,000 Daltons.

9. The process claimed in claim 8, wherein said concentration step and said optional diafiltration step are effected at a temperature of 2° to 65°C, preferably 50° to 60°C, or said extraction step, said separation step, said dilution step, said acidification step, said concentration step and said optional diafiltration step are effected at a temperature of 50° to 60°C.

10. The process claimed in claim 8 or claim 9, wherein said concentrated and diafiltered aqueous protein solution is dried to provide a soy protein product having a protein content of in excess of 60 wt% (N x 6.25) dry weight basis (d.b.)., preferably in excess of 90 wt%, more preferably at least 100 wt%.

11. The process claimed in claim 10, wherein a reducing agent is added to at least one of the soy protein source in the extraction step, the clarified aqueous soy protein solution following removal of residual soy protein source, the concentrated protein solution before or after diafiltration and/or is dry blended with dried soy protein product.

## Patentansprüche

1. Vorgang für die Herstellung einer Sojaproteinlösung, der Folgendes umfasst:
(a) Klassifizieren einer Sojaproteinquelle, um eine Sojaproteinquelle bereitzustellen, die weniger als 45 Gew.-%, bevorzugt weniger als 35 Gew.-%, an Partikeln, die klein genug sind, um ein 20-mesh-Sieb, angegeben in US-Siebgröße, zu passieren, enthält,
(b) Extrahieren der klassifizierten Sojaproteinquelle mit einer wässrigen Calciumchloridlösung, die eine Konzentration von 0,10 bis 0,15 mol/l(M) bei einem pH-Wert von 5 bis 11 aufweist, um eine Solubilisierung von Sojaprotein aus der klassifizierten Sojaproteinquelle zu bewirken und um eine wässrige Sojaproteinlösung auszubilden,
(c) Trennen der wässrigen Sojaproteinlösung von einer restlichen Sojaproteinquelle und entweder
(d) innerhalb von 20 Minuten von Schritt (c),
(i) Verdünnen der wässrigen Sojaproteinlösung auf eine Leitfähigkeit von weniger als 90 mS/cm (auch allgemein mS abgekürzt), bevorzugt 4 bis 18 mS/cm (auch allgemein mS abgekürzt), und
(ii) Einstellen des pH-Werts der wässrigen Sojaproteinlösung auf einen pH-Wert von 1,5 bis 4,4, bevorzugt 2 bis 4, um eine angesäuerte Sojaproteinlösung, die eine Extinktion von sichtbarem Licht bei 600 nm (A600) von weniger als 0,055, bevorzugt weniger als 0,040, aufweist, zu erzeugen, oder
(e) innerhalb von 40 Minuten von Schritt (c),
(i) Verdünnen der wässrigen Sojaproteinlösung auf eine Leitfähigkeit von weniger als 90 mS/cm (auch allgemein mS abgekürzt), bevorzugt 4 bis 18 mS/cm (auch allgemein mS abgekürzt),
(ii) Einstellen des pH-Werts der wässrigen Sojaproteinlösung auf einen pH-Wert von 1,5 bis 4,4, bevorzugt 2 bis 4, und
(iii) Wärmebehandeln der wässrigen Proteinlösung bei einer Temperatur von 70 °C bis 160 °C für 10 Sekunden bis 60 Minuten, um eine angesäuerte Sojaproteinlösung, die eine Extinktion von sichtbarem Licht bei 600 nm (A600) von weniger als 0,055 aufweist, zu erzeugen;
wobei der Vorgang ohne die Verwendung einer Feinfiltration durchgeführt wird.

2. Vorgang nach Anspruch 1, wobei die Extraktion der Sojaproteinquelle bei einer Temperatur von 1 °C bis 100 °C, bevorzugt 15 °C bis 65 °C, stärker bevorzugt 50 °C bis 60 °C bewirkt wird und/oder die Extraktion der Sojaproteinquelle bei einem pH-Wert von 5 bis 7 bewirkt wird und/oder die in dem Extraktionsschritt erzeugte wässrige Sojaproteinlösung eine Proteinkonzentration von 5 bis 50 g/L aufweist.

3. Vorgang nach Anspruch 1 oder 2, wobei der Trennschritt bei einer Temperatur von 1 °C bis 100 °C, bevorzugt 15 °C bis 65 °C, stärker bevorzugt 50 °C bis 60 °C, bewirkt wird oder der Extraktionsschritt und der Trennschritt bei einer Temperatur von 50 °C bis 60 °C bewirkt werden.

4. Vorgang nach einem der Ansprüche 1 bis 3, wobei die wässrige Sojaproteinlösung mit Wasser bei einer Temperatur von 1 °C bis 100 °C, bevorzugt 15 °C bis 65 °C, stärker bevorzugt 50 °C bis 60 °C, verdünnt wird oder der Extraktionsschritt, der Trennschritt, der Verdünnungsschritt und der Ansäuerungsschritt bei einer Temperatur von 50 °C bis 60 °C bewirkt werden.

5. Vorgang nach einem der Ansprüche 1 bis 4, wobei die Wärmebehandlung der wässrigen Proteinlösung bei einer Temperatur von 80 °C bis 120 °C für 10 Sekunden bis 5 Minuten, bevorzugt bei einer Temperatur von 85 °C bis 95 °C für 30 Sekunden bis 5 Minuten, bewirkt wird.

6. Vorgang nach einem der Ansprüche 1 bis 5, wobei die in Schritt (e) erzeugte angesäuerte wässrige Sojaproteinlösung zu einem Weiterverarbeiten auf eine Temperatur von 2 °C bis 65 °C, bevorzugt 50 °C bis 60 °C, abgekühlt wird.

7. Vorgang nach einem der Ansprüche 1 bis 6, wobei die angesäuerte wässrige Sojaproteinlösung getrocknet wird, um ein Sojaproteinerzeugnis bereitzustellen.

8. Vorgang nach einem der Ansprüche 1 bis 7, wobei die angesäuerte wässrige Sojaproteinlösung konzentriert wird, während die lonenstärke daraus im Wesentlichen konstant aufrechterhalten wird, um eine konzentrierte Sojaproteinlösung, die eine Proteinkonzentration von 50 bis 300 g/L, bevorzugt 100 bis 200 g/L, aufweist, bereitzustellen, bevorzugt unter Verwendung von Membranen, die eine Molekularmassentrenngrenze von 3.000 bis 1.000.000 einheitlicher atomarer Masseneinheiten (u = Dalton), bevorzugt 5.000 bis 100.000 Dalton, aufweisen, und die konzentrierte Sojaproteinlösung optional einer Diafiltration unter Verwendung von Wasser oder einer verdünnten Kochsalzlösung unterzogen wird, bevorzugt unter Verwendung von 1 bis 40 Volumina, bevorzugt 2 bis 25 Volumina, von Diafiltrationslösung, bevorzugt bewirkt, bis keine nennenswerten weiteren Mengen an Verunreinigungen oder sichtbare Farbe mehr in dem Permeat vorhanden sind oder bis das Retentat ausreichend gereinigt wurde, so dass, wenn getrocknet, es ein Sojaproteinisolat mit einem Proteingehalt von wenigstens 90 Gew.-% (N x 6,25) bereitstellt, unter Verwendung von Membranen, die eine Molekularmassentrenngrenze von 3.000 bis 1.000.000 Dalton, bevorzugt 5.000 bis 100.000 Dalton, aufweisen.

9. Vorgang nach Anspruch 8, wobei der Konzentrationsschritt und der optionale Diafiltrationsschritt bei einer Temperatur von 2 °C bis 65 °C, bevorzugt 50 °C bis 60 °C, bewirkt werden, oder der Extraktionsschritt, der Trennschritt, der Verdünnungsschritt, der Ansäuerungsschritt, der Konzentrationsschritt und der optionale Diafiltrationsschritt bei einer Temperatur von 50 °C bis 60 °C bewirkt werden.

10. Vorgang nach Anspruch 8 oder 9, wobei die konzentrierte und diafiltrierte wässrige Proteinlösung getrocknet wird, um ein Sojaproteinerzeugnis, das einen Proteingehalt von mehr als 60 Gew.-% (N x 6,25) Trockengewichtsbasis (dry weight basis - d.b.), bevorzugt von mehr als 90 Gew.-%, stärker bevorzugt wenigstens 100 Gew.-%, aufweist.

11. Vorgang nach Anspruch 10, wobei ein Reduktionsmittel zu wenigstens einer der Sojaproteinquellen in dem Extraktionsschritt, der geklärten wässrigen Sojaproteinlösung nach Entfernung der restlichen Sojaproteinquelle, der konzentrierten Proteinlösung vor oder nach der Diafiltration zugesetzt wird und/oder mit getrocknetem Sojaproteinerzeugnis trockengemischt wird.

## Revendications

1. Procédé de préparation d'une solution de protéines de soja, qui comprend :
(a) le classement d'une source de protéines de soja pour fournir une source de protéines de soja contenant moins de 45 % en poids, de préférence moins de 35 % en poids, de particules suffisamment petites pour passer par un tamis à 20 mailles, indiqué en taille de tamis des États-Unis,
(b) l'extraction de la source de protéines de soja classée au moyen d'une solution aqueuse de chlorure de calcium ayant une concentration de 0,10 à 0,15 mol/l(M) à un pH de 5 à 11 pour amener la solubilisation de protéines de soja à partir de la source de protéines de soja classée et pour former une solution aqueuse de protéines de soja,
(c) la séparation de la solution aqueuse de protéines de soja de la source de protéines de soja résiduelle, et soit
(d) dans les 20 minutes suivant l'étape (c), (i) la dilution de la solution aqueuse de protéines de soja à une conductivité inférieure à 90 mS/cm (également couramment abrégé mS), de préférence de 4 à 18 mS/cm (également couramment abrégé mS), et (ii) l'ajustement du pH de la solution aqueuse de protéines de soja à un pH de 1,5 à 4,4, de préférence de 2 à 4, pour produire une solution acidifiée de protéines de soja ayant une absorbance de la lumière visible à 600 nm (A600) inférieure à 0,055, de préférence inférieure à 0,040, ou
(e) dans les 40 minutes suivant l'étape (c), (i) la dilution de la solution aqueuse de protéines de soja à une conductivité inférieure à 90 mS/cm (également couramment abrégé mS), de préférence de 4 à 18 mS/cm (également couramment abrégé mS), (ii) l'ajustement du pH de la solution aqueuse de protéines de soja à un pH de 1,5 à 4,4, de préférence de 2 à 4, et (iii) le traitement thermique de la solution aqueuse de protéines à une température de 70 °C à 160 °C pendant 10 secondes à 60 minutes pour produire une solution acidifiée de protéines de soja ayant une absorbance de la lumière visible à 600 nm (A600) inférieure à 0,055 ;
le procédé étant exécuté sans l'utilisation de filtration fine.

2. Procédé revendiqué selon la revendication 1, dans lequel ladite extraction de la source de protéines de soja est effectuée à une température de 1 °C à 100 °C, de préférence de 15 °C à 65 °C, plus préférablement de 50 °C à 60 °C, et/ou ladite extraction de la source de protéines de soja est effectuée à un pH de 5 à 7 et/ou ladite solution aqueuse de protéines de soja produite dans l'étape d'extraction a une concentration en protéines de 5 à 50 g/L.

3. Procédé revendiqué selon la revendication 1 ou la revendication 2, dans lequel ladite étape de séparation est effectuée à une température de 1 °C à 100 °C, de préférence de 15 °C à 65 °C, plus préférablement de 50 °C à 60 °C, ou ladite étape d'extraction et ladite étape de séparation sont effectuées à une température de 50 °C à 60 °C.

4. Procédé revendiqué selon l'une quelconque des revendications 1 à 3, dans lequel ladite solution aqueuse de protéines de soja est diluée avec de l'eau à une température de 1 °C à 100 °C, de préférence de 15 °C à 65 °C, plus préférablement de 50 °C à 60 °C, ou ladite étape d'extraction, ladite étape de séparation, ladite étape de dilution et ladite étape d'acidification sont effectuées à une température de 50 °C à 60 °C.

5. Procédé revendiqué selon l'une quelconque des revendications 1 à 4, dans lequel ledit traitement thermique de la solution aqueuse de protéines est effectué à une température de 80 °C à 120 °C pendant 10 secondes à 5 minutes, de préférence à une température de 85 °C à 95 °C pendant 30 secondes à 5 minutes.

6. Procédé revendiqué selon l'une quelconque des revendications 1 à 5, dans lequel la solution aqueuse acidifiée de protéines de soja produite à l'étape (e) est refroidie pour un traitement supplémentaire à une température de 2 °C à 65 °C, de préférence de 50 °C à 60 °C.

7. Procédé revendiqué selon l'une quelconque des revendications 1 à 6, dans lequel la solution aqueuse acidifiée de protéines de soja est séchée pour fournir un produit de protéines de soja.

8. Procédé revendiqué selon l'une quelconque des revendications 1 à 7, dans lequel la solution aqueuse acidifiée de protéines de soja est concentrée tout en maintenant sa force ionique sensiblement constante pour fournir une solution concentrée de protéines de soja ayant une concentration en protéines de 50 à 300 g/L, de préférence de 100 à 200 g/L, de préférence à l'aide de membranes ayant un seuil de rétention des molécules de 3 000 à 1 000 000 d'unités de masse atomique unifiée (u = dalton) de préférence de 5 000 à 100 000 daltons, et la solution concentrée de protéines de soja est facultativement soumise à une diafiltration à l'aide d'eau ou d'une solution saline diluée, de préférence à l'aide d'1 à 40 volumes, de préférence de 2 à 25 volumes, de solution de diafiltration, de préférence effectuée jusqu'à ce qu'aucune quantités significatives supplémentaires de contaminants ou de couleur visible ne soit présente dans le perméat ou jusqu'à ce que le rétentat ait été suffisamment purifié de manière à, une fois séché, fournir un isolat de protéines de soja avec une teneur en protéines d'au moins 90 % en poids (N x 6,25), à l'aide de membranes ayant un seuil de rétention des molécules de 3 000 à 1 000 000 daltons, de préférence de 5 000 à 100 000 daltons.

9. Procédé revendiqué selon la revendication 8, dans lequel ladite étape de concentration et ladite étape de diafiltration facultative sont effectuées à une température de 2 °C à 65 °C, de préférence de 50 °C à 60 °C, ou ladite étape d'extraction, ladite étape de séparation, ladite étape de dilution, ladite étape d'acidification, ladite étape de concentration et ladite étape de diafiltration facultative sont effectuées à une température de 50 °C à 60 °C.

10. Procédé revendiqué selon la revendication 8 ou la revendication 9, dans lequel ladite solution aqueuse de protéines concentrée et diafiltrée est séchée pour fournir un produit de protéines de soja ayant une teneur en protéines supérieure à 60 % en poids (N x 6,25) sur la base du poids sec (d.b), de préférence supérieure à 90 % en poids, plus préférablement d'au moins 100 % en poids.

11. Procédé revendiqué selon la revendication 10, dans lequel un agent réducteur est ajouté à au moins l'une parmi la source de protéines de soja dans l'étape d'extraction, la solution aqueuse clarifiée de protéines de soja après élimination de la source de protéines de soja résiduelle, la solution de protéines concentrée avant ou après diafiltration et/ou est mélangé à sec avec un produit de protéines de soja séché.
